(19) Europäisches Patentamt
European Patent Office
Office européen des brevets

(11) **EP 3 038 660 B1**

(12) **EUROPEAN PATENT SPECIFICATION**

(45) Date of publication and mention
of the grant of the patent:
**23.12.2020 Bulletin 2020/52**

(21) Application number: **14839639.3**

(22) Date of filing: **27.08.2014**

(51) Int Cl.:
*A61K 47/61* (2017.01)     *A61K 31/445* (2006.01)
*A61K 31/573* (2006.01)     *A61K 31/415* (2006.01)
*A61K 31/635* (2006.01)     *A61K 31/145* (2006.01)
*A61K 31/18* (2006.01)     *A61K 31/454* (2006.01)
*A61K 31/7068* (2006.01)     *A61K 31/728* (2006.01)
*A61P 19/02* (2006.01)     *A61P 37/00* (2006.01)
*A61P 29/00* (2006.01)     *A61K 47/36* (2006.01)

(86) International application number:
**PCT/US2014/052813**

(87) International publication number:
**WO 2015/031425 (05.03.2015 Gazette 2015/09)**

(54) **COMPOUND OF GLYCOSAMINOGLYCAN AND ITS PREPARATION METHOD AS WELL AS APPLICATION**

VERBINDUNG VON GLYCOSAMINOGLYKAN UND DEREN HERSTELLUNGSVERFAHREN SOWIE ANWENDUNG

COMPOSÉ DE GLYCOSAMINOGLYCANE, PROCÉDÉ DE PRÉPARATION ET APPLICATION DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.08.2013 US 201361871352 P**

(43) Date of publication of application:
**06.07.2016 Bulletin 2016/27**

(73) Proprietor: **Aihol Corporation**
**Las Vegas, NV 89148 (US)**

(72) Inventor: **LIN, Hua-Yang**
**Taipei (TW)**

(74) Representative: **Viering, Jentschura & Partner mbB**
**Patent- und Rechtsanwälte**
**Hamborner Straße 53**
**40472 Düsseldorf (DE)**

(56) References cited:
**EP-A1- 1 082 963     EP-A2- 2 497 785**
**US-A1- 2004 157 810     US-A1- 2010 210 509**

**US-B2- 8 088 916**

• ONISHI HIRAKU ET AL: "In vivo evaluation of chondroitin sulfate-glycyl-prednisolone for anti-arthritic effectiveness and pharmacokinetic characteristics", INTERNATIONAL JOURNAL OF PHARMACEUTICS, vol. 456, no. 1, 14 August 2013 (2013-08-14), pages 113-120, XP028717504, ISSN: 0378-5173, DOI: 10.1016/J.IJPHARM.2013.08.007

• ITO ET AL: "Anti-inflammatory function of an in situ cross-linkable conjugate hydrogel of hyaluronic acid and dexamethasone", BIOMATERIALS, ELSEVIER SCIENCE PUBLISHERS BV., BARKING, GB, vol. 28, no. 10, 23 January 2007 (2007-01-23), pages 1778-1786, XP005856374, ISSN: 0142-9612, DOI: 10.1016/J.BIOMATERIALS.2006.12.012

• PAYAN E ET AL: "In vitro drug release from HYC 141, a corticosteroid ester of high molecular weight hyaluronan", JOURNAL OF CONTROLLED RELEASE, ELSEVIER, AMSTERDAM, NL, vol. 34, no. 2, 1 May 1995 (1995-05-01), pages 145-153, XP004037512, ISSN: 0168-3659, DOI: 10.1016/0168-3659(95)00002-P

**(Cont. next page)**

- JIN, Y.-J. ET AL.: 'Hyaluronic acid in drug delivery systems' JOURNAL OF PHARMACEUTICAL INVESTIGATION vol. 40, 2010, pages 33 - 43, XP055240494
- HUH, Y. ET AL.: 'Preparation and evaluation of spray-dried hyaluronic acid microspheres for intranasal delivery of fexofenadine hydrochloride' EUROPEAN JOURNAL OF PHARMACEUTICAL SCIENCES vol. 40, 2010, pages 9 - 15, XP055240497

## Description

CROSS REFERENCE

This application claims priority to U.S. Provisional Patent Application Serial No.61/871,352, filed on August 29, 2013.

## BACKGROUND OF THE INVENTION

1. Field of the Invention

The present invention relates to a compound consisting of a glycosaminoglycan conjugate with a drug according to the claims.

2. Description of the Prior Arts

The extracellular matrix (ECM) is a dynamic assemblage of interacting molecules that regulate cell functions and interactions in response to stimulation. One class of extracellular matrix macromolecules, the glycosaminoglycans, are molecules known to be involved in a wide array of both normal and abnormal biological processes, including cell migration, differentiation, proliferation, immune response and cytoskeletal organization.

Glycosaminoglycans (GAGs) are unbranched chains composed of repeating disaccharide units. These disaccharide units always contain an amino sugar (N-acetylglucosamine or N-acetylgalactosamine), which in most cases is sulfated, with the second sugar usually being an uronic acid (glucuronic or iduronic). GAGs are highly negatively charged because of the presence of carboxyl or sulfate groups on most of their sugar residues. As such they are strongly hydrophilic. GAGs tend to adopt highly extended conformations and form matrices that are space filling and resistant to compressive forces. Four main groups of GAGs have been distinguished by their sugar residues, the type of linkage between these residues, and the number and location of sulfate groups. They include: (1) hyaluronan, (2) chondroitin sulphate and dermatan sulfate, (3) heparan sulfate and heparin, and (4) keratan sulfate.

Hyaluronan (also called hyaluronic acid or hyaluronate or HA) is the simplest of GAGs. It consists of a regular repeating sequence of non-sulfated disaccharide units, specifically N-acetylglucosamine and glucuronic acid. Its molecular weight can range from 400 daltons (the disaccharide) to over millions of daltons. It is found in variable amounts in all tissues, such as the skin, cartilage, and eye, and in most, if not all, fluids in adult animals. It is especially abundant in early embryos. In articular cartilage, HA can form a large aggregate which is important for the function of cartilage. Furthermore, cell motility and immune cell adhesion is mediated by the cell surface receptor RHAMM (Receptor for Hyaluronan-Mediated Motility) and CD44.

HA is synthesized directly at the inner membrane of the cell surface with the growing polymer extruded through the membrane to the outside of the cell as it is being synthesized. Synthesis is mediated by a single protein enzyme, hyaluronan synthetase (HAS). By contrast, other GAGs are synthesized inside the cell in the Golgi apparatus, possibly in association with some core protein, and then released by exocytosis. HA degradation in vertebrate tissues *in vivo* is mediated by hyaluronidase, and exoglycosidases that remove sugars sequentially. Mammalian-type hyaluronidases have both hydrolytic and transglycosidase activities and can degrade HA and chondroitin. In connective tissue, the water of hydration associated with HA creates spaces between tissues, thus creating an environment conducive to cell movement and proliferation. HA plays a key role in biological phenomena associated with cell motility including rapid development, regeneration, repair, embryogenesis, embryological development, wound healing, angiogenesis, and tumorigenesis.

CD44 (also known as Pgp-1, Hermes-3, HCAM, ECMR III) is a widely expressed glycoprotein with a molecular weight of 85 to 90 kDa. CD44 is a major cell surface receptor for the glycosaminoglycan, hyaluronic acid (HA). CD44 binds HA specifically, although certain chondroitin-sulfate containing proteoglycans may also be recognized. CD44 plays a role in various cellular and physiological functions, including adhesion to and migration on HA, HA degradation and tumor metastasis. CD44 has also been shown to play a role in extracellular matrix binding, cell migration, lymphocyte activation, lymphocyte homing, and proliferation of bronchial smooth muscle cell (Gunthert et al., 1991, A new variant of glycoprotein CD44 confers metastatic potential to rat carcinoma cells, 5;65(1): 13-24). The CD44 receptor shows a complex pattern of alternative splicing in its variable region of the extracellular domain. CD44 appears to be a particularly important leukocyte receptor for HA and may therefore have a role in the pathogenesis of asthma. In addition, levels of HA, which were increased during experimental asthma in control mice were markedly attenuated in the antibody-treated mice, supporting a role for CD44 in HA metabolism (specifically in the breakdown of high molecular weight HA to pro-inflammatory low molecular weight forms). This may be particularly important because HA-derived oligosaccharides can bind and activate Toll-like receptor. Clearly, the most impressive aspect of the results is the profound magnitude of the beneficial effects of anti-CD44 treatment.

HA-CD44 interactions may play an important role in development, inflammation, T cell recruitment and activation, lung inflammation, and tumor growth and metastasis.

Mice with a targeted deletion of standard CD44 and all isoforms develop normally. Studies have suggested an important role for CD44 in inflammatory states such as rheumatoid arthritis and the extravasation of T cells to sites of tissue inflammation. CD44 may have an important role in the recruitment of inflammatory cells in allergen-induced airway inflammation in mice. CD44 has been suggested to play a critical role in regulating chronic inflammation, suggesting that CD44 may have a critical role in regulating macrophage activation independently of interactions with HA (Dianhua Jiang, Hyaluronan in Tissue Injury and Repair, Annu. Rev. Cell Dev. Biol. 2007;23:435-61).

The ability to control inflammatory and immune responses is central to the therapy of a wide spectrum of diseases. CD44 supports the adhesion of activated lymphocytes to endothelium and smooth muscle cells. Furthermore, ligation of CD44 induces activation of both inflammatory and vascular cells. HA, the principal ligand for CD44, is upregulated in atherosclerotic lesions of apoE-deficient mice and the low-molecular-weight proinflammatory forms of HA stimulate VCAM-1 (vascular cell adhesion molecule-1) expression and proliferation of cultured primary aortic smooth muscle cells, whereas high-molecular-weight forms of HA inhibit smooth muscle cell proliferation. Gal-9 (Galectin-9) can reduce AHR (airway hyperresponsiveness) as well as Th2-associated airway inflammation. Furthermore, administration of Gal-9 as well as anti-CD44 monoclonal antibody inhibited the infiltration of peripheral blood Th2 cells into the airway. Interestingly, Gal-9 directly bound the CD44 adhesion molecule and inhibited interactions with HA. Consistent with the concept that CD44-HA interactions mediate the migration of T cells into the lung, Gal-9 blocked CD44-dependent adhesion of BW5147 mouse T cells to HA. It was concluded that Gal-9 inhibits allergic inflammation of the airway and AHR by modulating CD44-dependent leukocyte recognition of the extracellular matrix (Shigeki Katoh, et al., Galectin-9 Inhibits CD44-Hyaluronan Interaction and Suppresses a Murine Model of Allergic Asthma, American Journal of Respiratory and Critical Care Medicine, 2007 Jul 1;176(1):27-35).

The CD44 will be expression at the autoimmune disease likes systemic lupus erythematosus (SLE), Rheumatoid arthritis, Sjögren's syndrome, inflammatory bowel disease (IBD), Ankylosing spondylitis, Psoriatic arthritis, Psoriasis, Dermatomyosistis, Vasculitis, and Behcet's disease. Systemic lupus erythematosus is a prototype autoimmune disease that affects multiorgan systems. Accumulating evidence suggests that hyaluronan and its interaction with its cell surface receptor CD44 plays an important role in mediating pathogenic mechanisms in SLE (Yung S and Chan TM, 2012, The Role of Hyaluronan and CD44 in the Pathogenesis of Lupus Nephritis, Autoimmune Dis. Volume 2012 (2012), Article ID 207190, 9 pages). Rheumatoid Arthritis (RA) is a common autoimmune disorder that results in inflammation of the synovial joints of patients. Though RA affects approximately 1% of the population and is classified as an autoimmune disorder, the molecular event(s) which initiate the evasion of tolerance remain speculative and unconfirmed (Patrick J. Mott, CD44 Antibodies and Immune Thrombocytopenia in the Amelioration of Murine Inflammatory Arthritis, PLoS One, 2013, 8(6): e65805). While Sjögren's syndrome (SS) is more common than related autoimmune disorders, such as systemic lupus erythematosus (SLE) and rheumatoid arthritis (RA), scientific and medical research in SS has lagged behind significantly. This is especially true in the field of SS genetics, where efforts to date have relied heavily on candidate gene approaches (John A. Ice, Genetics of Sjögren's syndrome in the genome-wide association era, J Autoimmun. 2012 Aug;39(1-2):57-63). Ankylosing Spondylitis (AS) is a common inflammatory rheumatic disease with a predilection for the axial skeleton, affecting 0.2% of the population. Current diagnostic criteria rely on a composite of clinical and radiological changes, with a mean time to diagnosis of 5 to 10 years (Roman Fischer, 2011, Discovery of Candidate Serum Proteomic and Metabolomic Biomarkers in Ankylosing Spondylitis, Mol Cell Proteomics, 2012 Feb;11(2):M111.013904). The results suggest that circulating T lymphocytes bearing activated CD44 are elevated under conditions of chronic inflammation and that these may represent a pathogenically important subpopulation of activated circulating cells that may provide a reliable marker for autoimmune or chronic inflammatory disease activity (Estess P, et al., 1998, Functional activation of lymphocyte CD44 in peripheral blood is a marker of autoimmune disease activity, J Clin Invest. 1998 Sep 15;102(6):1173-82). Inflammatory bowel disease (IBD), including Crohn's disease (CD) and ulcerative colitis (UC), shares clinical and immunological features with psoriasis. Genome-wide association studies have found common susceptibility genes. However, epidemiologic data evaluating the association between psoriasis, psoriatic arthritis and risk of IBD are sparse. This research aimed to evaluate the association between psoriasis, psoriatic arthritis and incident CD and UC among women in the USA. Psoriasis with concomitant psoriatic arthritis is associated with an increased risk of incident CD (Wen-Qing Li, 2013, Psoriasis, psoriatic arthritis and increased risk of incident Crohn's disease in US women, Ann Rheum Dis. 2013 Jul;72(7):1200-5). Dermatomyositis (DM) is a connective-tissue disease related to polymyositis (PM) that is characterized by inflammation of the muscles and the skin. While DM most frequently affects the skin and muscles, it is a systemic disorder that may also affect the joints, the esophagus, the lungs, and, less commonly, the heart. Vasculitis is a group of disorders that destroy blood vessels by inflammation. Both arteries and veins are affected. The pathophysiology of vasculitis is not well understood, but the ensuing inflammatory response has been generally well described (Henry S. Su, 2012, Vasculitis: Molecular Imaging by Targeting the Inflammatory Enzyme Myeloperoxidase, Radiology, 2012 Jan;262(1): 181-90). Behcet's disease (BD) is the only systemic vasculitis involving both arteries and vein in any sizes. It is frequently encountered in rheumatology clinics. It has some major morbidities and even fatal outcomes in some cases (M. B. Owlia, 2012, Behcet's Disease: New Concepts in Cardiovascular Involvements and Future Direction for Treatment, ISRN Pharmacol, 2012: 760484).

Interferon alpha (IFNα) conjugated with polyethylene glycol (PEG) has been widely used for the treatment of hepatitis C virus (HCV) infection as a once-a-week injection formulation. However, the PEGylated IFNα has a low efficacy of 39% and a side effect after repeated injections possibly due to the nonspecific delivery with PEGylation. Therefore, target specific long-acting hyaluronic acid-interferon alpha (HA-IFNα) conjugate was developed for the treatment of HCV infection. HA-IFNα conjugate was synthesized by coupling reaction between aldehyde modified HA and the N-terminal group of IFNα. The IFNα content could be controlled in the range of 2-9 molecules per single HA chain with a bioconjugation efficiency higher than 95%.

Several studies have implicated CD44 in direct interactions between bacteria and host cells, as well as in signaling events that alter host cells and make them more susceptible to infection. Streptococcus pyogenes, for example, attaches to cells through its hyaluronan-rich polysaccharide capsule (or cell wall). HA binds to CD44, which in turn triggers tyrosine phosphorylation of several host-cell proteins, as well as cytoskeletal rearrangements that cause ruffles and the extension of lamellipodia. As a result, the intercellular adhesion is loosened owing to a reduced number of tight junctions and of E-cadherin, a process that allows the entry of bacteria into subepithelial tissue.

WO94/09811 describes the use of CD44 in treating inflammation or detecting cancer metastasis. The authors show that CD44 is upregulated in inflammatory conditions and CD44 peptides are capable of inhibiting T-cell activation. No data or claims are presented on inhibition of metastasis by CD44 and no claims are made towards use of CD44 for inhibiting tumor growth or angiogenesis. WO 99/45942 discloses the use of HA-binding proteins and peptides including CD44 to inhibit cancer and angiogenesis-dependent diseases. This publication uses metastatin, a 38 kDa fragment of the cartilage link protein, as well as a HA-binding peptide derived from this fragment to inhibit pulmonary metastasis of B 16 mouse melanoma and Lewis lung carcinoma. In the case of the HA-binding peptide, growth of B 16 melanoma on chicken CAM and endothelial cell migration on HA have been inhibited. In both publications the use of HA-binding peptides is directly related to their ability to bind hyaluronic acid.

US patent No. 8,192,744 shows that soluble recombinant CD44 hyaluronic acid binding domain (CD44HABD) inhibits angiogenesis in vivo in chick and mouse and thereby inhibits human tumor growth of various origins. The invention discloses soluble non glycosylated CD44 recombinant proteins as a novel class of angiogenesis inhibitors based on targeting of vascular cell surface receptor.

Thus, the prior art discloses the potential use of CD44 to specify that any effects are dependent on HA-CD44-interaction. Consequently, all utility ascribed this far to CD44-HA conjugate is directly dependent on their ability to bind hyaluronic acid.

However, some drugs are still not successfully conjugated onto HA and further experiments should be carried out to confirm the potential usefulness of HA as site-delivery carrier of active compound. In particular, the prior art has not shown that the interactions between the surface cell receptor CD44 and a conjugate of HA with an active compound can be profitably exploited for a target delivery of such active compound in diseases characterized by an overexpression of CD44 obtaining an effective therapeutic improvement of the same.

Onishi Hiraku et al., In vivo evaluation of chondroitin sulfate-glycyl-prednisolone for anti-arthritic effectiveness and pharmacokinetic characteristics, International journal of pharmaceutics, 2013, 456(1):113-120, discloses a conjugate between chondroitin sulfate and glycyl-prednisolone for the treatment of rheumatoid arthritis.

US2004/157810 discloses conjugates of corticosteroids to bulky groups such as hyaluronic acid.

## SUMMARY OF THE INVENTION

The purpose of the present invention is to provide new compound based on the conjugation of HA with active compound suitable for a site delivery of such active compound in diseases overexpressing the surface cell receptor CD44.

The present invention, therefore, provides a compound conjugating glycosaminoglycan with a drug, wherein the drug is used for treating diseases of inflammation that are highly related with the expression of CD44.

In a first aspect, it is an object of the invention to provide a compound consisting of a conjugate from a glycosaminoglycan and an active compound, wherein the active compound is conjugated by means of a functional group to a carboxylic group of the glycosaminoglycan or a salt thereof to form a covalent conjugation, and wherein the active compound is selected from a group consisting of fexofenadine, budesonide succinate, and prednisolone succinate.

The glycosaminoglycan of the conjugate according to the present invention is preferably hyaluronic acid.

Furthermore, the glycosaminoglycan conjugate according to the present invention is preferably for use for treating inflammation diseases.

Therefore, in a second aspect it is a further object of the invention to provide a compound consisting of a conjugate from a glycosaminoglycan and an active compound, wherein the active compound is conjugated by means of a functional group to a carboxylic group of the glycosaminoglycan or a salt thereof to form a covalent conjugation, and wherein the active compound is selected from a group consisting of fexofenadine, budesonide succinate, and prednisolone succinate for use in the treatment of inflammation and for the preparation of pharmaceutical compositions for said therapeutic treatment.

Also disclosed herein is a method for preparing a compound consisting of a conjugate from a glycosaminoglycan and an active compound, wherein the active compound is conjugated by means of a functional group to a carboxylic group of the glycosaminoglycan or a salt thereof to form a covalent conjugation, and wherein the active compound is selected from a group consisting of fexofenadine, budesonide succinate, and prednisolone succinate.

## BRIEF DESCRIPTION OF THE DRAWINGS

To adequately describe the present invention, references to embodiments thereof are illustrated in the appended drawings. These drawings herewith form a part of the specification.

Fig. 1 shows the affinity of hyaluronic acids (HAs) by fluorescent index in normal and injured colon tissues;
Fig. 2 shows the fluorescence results of HA-dye compound working on HCT 15 cell line and HT29 cell line with different time course, wherein Fig 2A represents HCT 15 cell line at 6 hours; Fig.2B represents HCT 15 cell line at 12 hours; Fig 2C represents HT29 cell line at 6 hours; Fig.2D represents HT29 cell line at 12 hours.
Fig. 3 shows the structure of HA-Celecoxib conjugate.
Fig. 4 shows the anti-inflammation effect of Control, lipopolysaccharide (LPS), HA, Celecoxib (Cele), and HA-Celecoxib conjugate (HA-cele) on Raw 264.7 cell line. The inflammation index herein is change percentage of prostaglandin $E_2$ ($PGE_2$) amount.
Fig. 5 shows the structure of HA-ADH-Budesonide conjugate.
Fig. 6 shows the anti-inflammation effect of Control (ctrl), LPS, HA, HA plus LPS (HA+LPS), Budesonide (bude.), Budesonide plus LPS (bude+LPS), HA-Budesonide conjugate (HA-bude, "HA-bude" stands for "HA-ADH-Budesonide") and HA-Budesonide conjugate plus LPS (HA-bude+LPS) on Raw 264.7 cell line. The inflammation index herein is change of nitrite (NO) concentration.
Fig. 7 shows the structure of HA-ADH-Fexofenadine conjugate.
Fig. 8 shows the anti-inflammation effect of Control, LPS, HA, Fexofenadine (Fexo), and HA-Fexofenadine conjugate (HA-fexo, "HA-fexo" stands for "HA-ADH-Fexofenadine") on Raw 264.7 cell line. The inflammation index herein is change percentage of prostaglandin $E_2$ ($PGE_2$) amount.
Fig. 9 shows the structure of HA-ADH-Prednisolone conjugate.
Fig. 10 shows the anti-inflammation effect of Control, LPS, HA, Prednisolone (Pred), and HA-Prednisolone conjugate (HA-pred, "HA-pred" stands for "HA-ADH-Prednisolone") on Raw 264.7 cell line. The inflammation index herein is change percentage of prostaglandin $E_2$ ($PGE_2$) amount.
Fig. 11 shows treatment effect of Rheumatoid arthritis (RA) by control (vehicle), Prednisolone (Pred), HA conjugated with Prednisolone (HA-ADH-Pred), and HA. The treatment index herein is change percentage of paw thickness (mean$\pm$SE). Rats were fed for inducing RA during D1 to D13, and dosing on D14. Data were calculated by Non-parametric statistics, $*p<0.05$ vs. Vehicle group; $^{\#}p<0.05$ vs. Prep group and $^{\&}p<0.05$ vs. HA group.
Fig. 12 shows treatment effect of Rheumatoid arthritis (RA) by control (vehicle), Prednisolone (Pred), HA conjugated with Prednisolone (HA-ADH-Pred), and HA. The treatment index herein is change percentage of ankle circumference (mean$\pm$SE). Rats were fed for inducing RA during D1 to D13, and dosing on D14. Data were calculated by Non-parametric statistics, $*p<0.05$ vs. Vehicle group; $^{\#}p<0.05$ vs. Prep group and $^{\&}p<0.05$ vs. HA group.

## DETAILED DESCRIPTION OF THE PREFERRED EMBODIMENTS

Other objectives, advantages and novel features of the invention will become more apparent from the following detailed description when taken in conjunction with the accompanying drawings.

In general, a drug orally administered or injected into circulation system must directly arrive at its targeted treatment area, and therefore the drug effect on targeted disease and normal organ is very similar because the concentration and specificity are not so high on the targeted site, limited by safety profile.

In order to improve the therapy efficacy combining the same with good safety profile, one strategy is to modify the drug to be more target-selective to the disease area through a covalent binding the drug with a carrier. This is a need particularly felt in the field of anti-inflammatory therapy as previously anticipated.

At this purpose, the inventor has conceived the idea to exploit the interactions between the hyaluronic acid (HA) and its receptor CD44 for a target delivering active substance.

The idea to maintain the relative higher concentration of the drug on the targeted site versus normal tissue or organ has been established by the inventor following long-term study and experiment on HA.

The results, from which the present invention originates, are fully described in the examples and are hereinafter briefly summarized.

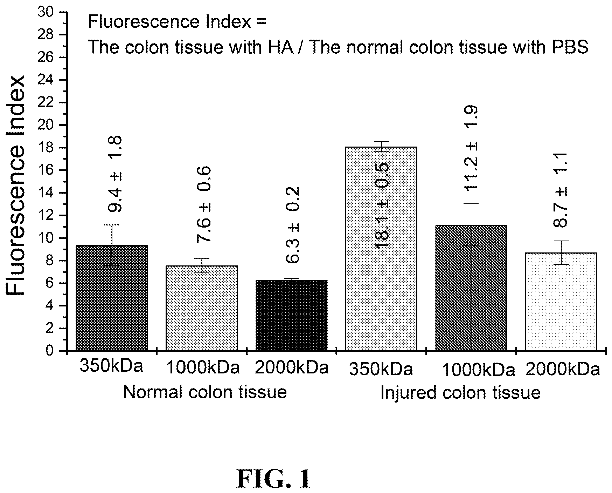

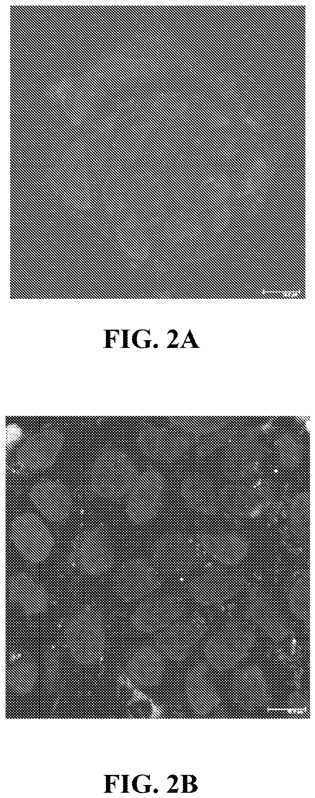

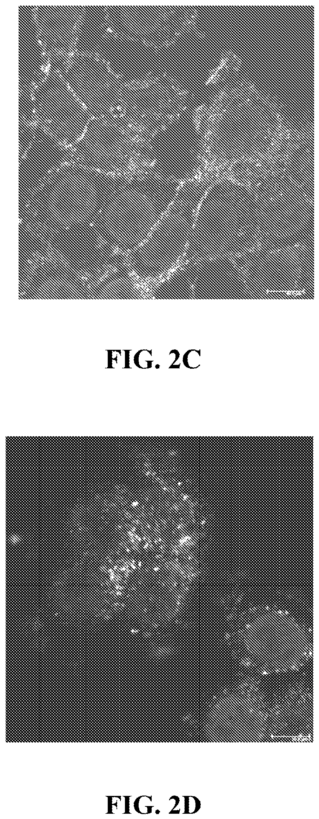

In fact, the present invention finds support on the result showing that hyaluronic acids having different average molecular weights (MW) have an adhesion index higher in injured tissue than in normal tissue and that the HA with low average molecular weight performs better than the HAs with high average molecular weights. In particular, as shown in Fig. 1, comparing the differences among HAs of three average molecular weights adhered on the injured colon tissues, the fluorescent index of adhesion of 350 KDa HA by the injured colon tissues was higher than the HAs of the other two average molecular weights (2000 KDa = 2 MDa) and (1000 KDa = 1 MDa). Further, the fluorescent index of adhesion of 1 MDa HA by even normal or injured colon tissues was higher than 2 MDa HA. This result confirmed that the HA can more specifically adhere on the inflammation site, which induces the inventor to further invent the present invention and to verify whether this peculiar feature of tissue adhesion of hyaluronic acid, allegedly due to an interaction of HA with its surface cell receptor CD44, can be maintain when this glycosaminoglycan is conjugated with other compounds. Therefore, the inventor further conjugates a drug with HA in order to verify if HA can be used as a targeted delivery vehicle to conduct the drug onto CD44 abundance site. As aforementioned, when CD44 is overexpressed during the situation of existence of inflammation, infection or a cancer, a related drug can easily arrive at and retain relative high concentration on the targeted site owing to ligand HA attaches onto receptor CD44. In accompany with HA's adherence effect to inflammation site or CD44 abundance site, the conjugated drug should especially aggregate on the targeted part to enhance the therapy efficacy owing to relative higher concentration of the drug on the site, and hence decreasing accordingly the amount of the drug utilized with better safety profile. In order to confirm the drug or dye has been successfully conjugated with HA and further confirm the HA attachment effect, the inventor of the present invention conducted an experiment including conjugating dye onto HA (HA-dye) and treating with the cell lines and mice separately. Fig. 2A and Fig. 2B show the experiments at different working times on cell line HCT15 (a colorectal adenocarcinoma with less CD44), and Fig. 2C and Fig. 2D show the experiments at different working times on cell line HT29 (a colorectal adenocarcinoma with rich CD44). The results of HT29 (Fig. 2C and Fig. 2D) show the HA-dye have been successfully conjugated and attached onto CD44 abundant area of HT29 (Fig. 2C), and even enter into HT29 cells (Fig. 2D). That means the idea of the present invention is proper and effective and also means drug or dye can be conjugated to HA and that HA retain its capability to bind CD44.

The attachment condition of free dye and HA-dye on cell lines of HT29 and HCT15 of mice for 4 weeks was conducted. The free dye was injected into the tail vein of the mice. The result showed that the two different CD44 expression cancer cells without any difference in attachment result. The ratio of attachment area of HT29 is 50.15%, whereas HCT15 is 49.86%. However, when the HA conjugated dye was injected into the mice tail vein, the more CD44 expression cancer cell HT 29 showed significant concentration of HA conjugated dye, but the less CD44 expression HCT15 showed very limited result. The ratio of attachment area of HT29 is 74.15%, whereas HCT15 is 25.85%. The result can show that when dye conjugated with HA, the concentration of dye was increased owing to HA attached on CD44 abundant site.

CD44 highly related diseases include cancer, infection and inflammation. Infectious pathogens include, but not limited to, some viruses, bacteria, fungi, protozoa, multicellular parasites, and aberrant proteins known as prions. These pathogens are the cause of disease epidemics, in the sense that without the pathogen, no infectious epidemic occurs. In a preferred embodiment, infection disease includes lower respiratory infections, HIV/AIDS, diarrheal diseases, tuberculosis, malaria, measles, pertussis, tetanus, meningitis, syphilis, hepatitis B, sepsis, and tropical diseases. Inflammatory abnormalities are a large group of disorders which underlie a vast variety of human diseases. In a preferred embodiment, inflammation disease includes acne vulgaris, allergic diseases, asthma, autoimmune diseases, celiac disease, chronic prostatitis, glomerulonephritis, hypersensitivities, inflammatory bowel diseases, pelvic, inflammatory disease, reperfusion injury, rheumatoid arthritis, sarcoidosis, transplant rejection, vasculitis, and interstitial cystitis.

In the specification the term "drug" or "compound" or "agent" may comprise anti-asthma drug, anti-histamine drug, anti-infection drug, anti-inflammatory drug, anti-allergy drug, anti-virus drug, immunosuppressant, NSAID (Non-Steroidal Anti-Inflammatory Drug), and steroid. Among them, anti-allergy drug, anti-infection drug, anti-inflammatory drug, and steroid are preferred. The majority of anti-allergy drugs can be divided into anti-histamines, anti-inflammatory agents, decongestants and steroid such as Fexofenadine, cetirizine, chlorpheniramine maleate, pseudoephedrine, and Budesonide. The majority of anti-infection drugs can be divided into local anti-infection means that are used externally on skin or mucous membranes without entering the blood stream and metabolizing in the liver; and systemic anti-infection means which are used to treat internal organ infections through oral or injected administration. The majority of anti-inflammatory drugs can be divided into non-steroidal anti-inflammatories or NSAIDs, COX-2 antagonist and steroid.

Examples of drugs are anti-asthma drug, anti-fungal drug, anti-histamine drug, anti-inflammatory drug, anti-virus drug, NSAID and steroid thereof. Disclosed herein is the conjugation of anti-asthma drug including Salbutamol; NSAID including Nimesulide, Celecoxib, Meloxicam, Diclofenac and Piroxicam; anti-allergy drug including Fexofenadine; anti-fungal drug including Amphotericin B; anti-virus drug including Ribavarin; and steroid including Budesonide and Prednisolone to HA Further, COX-2 antagonist includes Celecoxib.

The aim of the present invention is binding or conjugating HA with a drug according to the claims, with or without a linker or spacer, by carboxyl group, hydroxyl group, or amino group of HA to accomplish working effect on specific location and specific time. Therefore, HA as a target delivery vehicle to carry the drug to the specific site that has abundant CD44 can produce better treatment efficacy and safety.

As used herein, in general, the term "linker" or "spacer" means an organic moiety that connects two parts of a compound. Linkers typically comprise a direct bond or an atom such as oxygen or sulfur, a unit such as SS, NH, C(O), C(O)NH, SO, $SO_2$, $SO_2NH$ or a chain of atoms, such as substituted or unsubstituted alkyl where one or more methylenes can be interrupted or terminated by O, S, S(O), $SO_2$, NH, $NH_2$, C(O). The term "linker" or "spacer" of the present invention may be absent and denotes any chemical compound present between the drug and the HA which may be removed chemically, enzymatically or may decompose spontaneously; it also contains at least one other group useful for linking the drug, e.g. amino, thiol, further carboxyl groups, etc. The linker or spacer may be a polypeptide, a peptide, or a lipid.

Suitable linkers or spacers are e.g. adipic dihydrazide (ADH), linear or branched, aliphatic, aromatic or araliphatic $C_2$-$C_{20}$ dicarboxylic acids, amino acids, peptides.

The role of the linker, whenever present, consists in creating an arm or a spacer between the hyaluronic acid and the drug. The linker engages, on one side, the HA via the amide, carboxyl group, hydroxyl group, or amino group linkage and, on the other side, the drug via any possible covalent-type bond.

When the linker or spacer is a dicarboxylic acid, the carboxylic group forming the ester bond with the drug may be the hydroxyl group of the compound. When the linker or spacer is a dihydrazide, the amino group forming the amide bond with HA may be the free carboxylic group of the HA. Preferred linkers or spacers are: succinic acid to drug, adipic dihydrazide to HA.

The present invention provides a compound consisting of a conjugate from a glycosaminoglycan, preferably hyaluronic acid, and an active compound, wherein the active compound is conjugated by means of a functional group to a carboxylic group of the glycosaminoglycan or a salt thereof to form a covalent conjugation, and wherein the active compound consists of fexofenadine, budesonide succinate or prednisolone succinate.

In a preferred embodiment of the present invention, the covalent conjugation between one of the functional carboxyl groups of HA and of the active compound is a direct conjugation by an ester bond.

In case of indirect conjugation by means of a linker, said linkers are selected from a dihydrazide, adipic dihydrazide, polypeptide, a peptide, a lipid, an aminoacid or a linear or branched, aliphatic, aromatic or araliphatic $C_2$-$C_{20}$ dicarboxylic acids.

The preferred HA for conjugation has an average molecular weight in the range comprised from 5 kDa to 2000 kDa and the conjugation involves at least 40 % of the carboxyl group of HA.

In order to treat the acute or chronic inflammation, where the acute or chronic inflammation is induced or caused by infection, injury, autoimmune disease, or allergy and the autoimmune disease including Rheumatoid arthritis, the preferred embodiment of the formulation or dosage form of the present invention including an excipient and/or diluent to formulate an administrating dosage form for oral or circulation system use. The more preferred embodiment of the oral dosage form is selected from the group consisting of solid dosage form, solution including, but not limited to suspension, tablet including, but not limited to controlled-release tablet, and capsule including, but not limited to enteric-coated capsule. The more preferred embodiment of the circulation system or systemic administration form is selected from the group consisting of intro-venous (IV), intra-muscle (IM) and subcutaneous (SC).

The following examples are given for the purpose of illustrating various embodiments of the invention and are not meant to limit the present invention in any fashion.

## EXAMPLE

### EXAMPLE 1: The adhesion of HA in colon tissue (IVIS image system-vision 3)

Procedure:

1. 0.25 g high molecule weight sodium hyaluronate powder (HHA; Mw: 2 MDa; Freda) and 0.25 g low molecule weight sodium hyaluronate powder (LHA; Mw: 0.35 MDa; Freda) were added into 50 ml PBS buffer (Phosphate buffered saline) respectively to form 0.5% solution, and then stirred for 6 hours until the powder was totally dissolved. 0.25 g medium molecule weight sodium hyaluronate powder (MHA; Mw: 1 MDa; Freda) was added into 50 ml PBS buffer, and then stirred for 6 hours until the powder was totally dissolved and ready for use in the following steps.

2. Fluorescent HA (HA-f) was prepared by (1) 0.39 g MES free acid (2-(N-morpholino) ethanesulfonic acid, Calbiochem) and was dissolved in 100 ml dd water. (2) Solution A: 65 mg fluroresceinamine powder, (isomer I, Fluka) was dissolved in 9 ml 95% EtOH solution and then stirred for 10 minutes under a condition that light was prohibited. (3) Solution B: 359 mg EDC powder (N-(3-Dimethylamino propyl)-N-ethyl carbodiimide hydrochloride, Sigma) was dissolved in 9 ml MES buffer and then stirred for 10 minutes. (4) Solution C: 216 mg NHS powder (N-Hydroxysuccinimde, Sigma) was dissolved in 9 ml MES buffer and then stirred for 10 minutes. (5) 3 ml Solution A was slowly dropped into 50 ml 0.5% HA solution and then stirred for 10 minutes under a condition that light was prohibited. (6) 3 ml Solution B and 5 ml Solution C were separately dropped into the solution of step (5) and then stirred for 10 minutes under a condition that light was prohibited. (7) 0.02 M MES buffer was slowly added into the solution of step (6) until the volume reached 100 ml and was then stirred for 24 hours at room temperature under a condition that light was prohibited. (8) The product after reaction was poured into a dialysis tubing (MW: 12000~14000) in 5 L dd water as a dialysis solution and then stirred for 5 days at 4°C under a condition that light was prohibited with dialysis solution being changed every 12 hours until the dialysis solution had no fluorescence. (9) The liquid after dialysis was allocated into 50 c.c. plastic centrifuge tubes and then reserved at -20°C refrigerator overnight, followed by drying in a freeze-drying machine under a condition that light was prohibited. (10) The dried HA-f powder was reserved at -20°C refrigerator. (11) 50 mg HA-f powder was slowly added into 10 ml PBS buffer and then stirred for 6 hours until the powder was totally dissolved.

3. Colon tissue of SD-rat (Sprague-Dawley Rat) aged 7-8 weeks was cut by scalpel and then washed by PBS buffer, followed by being cut to 3-4 cm long with soaking in PBS buffer finally.

4. Injured colon tissue was prepared by brushing by toothbrush for 20 times longitudinally and then soaking in PBS buffer.

5. Normal and injured colon tissues were put into a 12-well plate and then 1 ml 0.5 % HA-f solution was added into each well and shaken for 2 hours at room temperature. Surplus HA-f solution was sucked by tip 2 hours later, and then soaked into PBS buffer for 10 minutes followed by removing PBS buffer repeatedly for 3 times.

6. Cleaned colon tissue was placed in a 12-well plate with lining tissue upwards and then placed onto the dock of the IVIS (in vivo image system, XENOGEN). The default parameter was set up as GFP (green fluorescent protein) whereas the excitation was 465 nm and the emission was 500 nm and then the image was captured by software.

7. All values are calculated as means of n observations. The histological index was analyzed by Student's t-test.

Result: The fluorescent index was quantified and arranged as in Fig. 1. The fluorescent index of normal colon tissue was defined as 1. The other colon tissues tests were calibrated by the defined value. The result showed that the HAs with the same average Mw were adhered in the injured colon tissues with obviously higher fluorescent index than in the normal colon tissues (P<0.01). Comparing the difference between HAs of three different average molecular weights adhered in the injured colon tissues, the fluorescent index of adhesion of 350 KDa HA by the injured colon tissues was obviously higher than HAs of the other two average molecular weights (2 MDa and 1 MDa). Further, the fluorescent index of adhesion of 1 MDa HA by even normal or injured colon tissues was higher than 2 MDa HA.

**EXAMPLE 2: HA-dye conjugation process and HA-dye *in vitro* image** (comparative example)

The following whole process of HA-dye conjugation must be kept in dark. The synthesis of HA-ADH

1. HA (0.34 MDa, 50 mg) was dissolved in water to give a concentration of 4 mg/ml.
2. 5-fold excess (114.8 mg) of ADH was added into the solution.
3. The pH of the reaction mixture was adjusted to 4.75 by addition of 0.1 N HCl.
4. Next, 1 equiv. (25.1 mg) of EDC was added in solid form. The pH of the reaction mixture was maintained at 4.75 by addition of 0.1 N HCl.
5. After 15 minutes reacting, the reaction was quenched by addition of 0.1 N NaOH to adjust the pH of reaction mixture to 7.0.
6. The reaction mixture was then transferred to pretreated dialysis tubing (Mw cutoff 3500) and dialyzed exhaustively against 100 mM NaCl, then 25% EtOH /water 4 cycles and finally water. The solution was then filtered through 0.2 $\mu$m cellulose acetate membrane, flash frozen, and lyophilized.
7. The substitution degree of ADH was measured by 1 H NMR.

The synthesis of HA-ADH-FITC

1. HA-ADH (DS=36%) 88 mg was dissolved in 35 ml water
2. FITC 9.5 mg was dissolved in 10 ml DMSO.
3. Mix HA-ADH solution and FITC solution
4. After stirred 48 h at room temperature, the solution was dialyzed 3 days with 0.3 M NaCl and pure water alternately using MWCO 12000-14000 dialysis bag.
5. The solution was then freeze-drying 2 days.
6. Finally the degree of substitution was determined by UV spectrum.

HA-dye *in vitro* image

(1) 1x10$^5$ HT 29 cells and HCT15 cells (human colon carcinoma, a CD44 positive cell) were seeded onto a microscope slide in a 3.5 cm dish.
(2) Indicated dye concentrations, 1 $\mu$M of HA-dye (HA: 0.34 MDa) were added into cells for indicated time respectively.
(3) After incubation, cells were washed in PBS, and then fixed in 3.7% formaldehyde.
(4) Observation of the interaction between HA-dye and cells was performed by confocal microscopy.

Result: The fluorescent view can show the attachment site and amount of dye on HCT15 (Fig.2A and Fig.2B) and HT29 (Fig.2C and Fig.2D). The results reveal that dye has been successfully conjugated with HA and HA enhances HA-dye concentration on CD44 abundant site on HT29, whereas HT29 has stronger fluorescence that meets with more abundant CD44 than HCT15 has. Even proved that HA-dye can enter the cells (Fig.2D). The HA-dye was accumulated after a 6 hours treatment and internalized after a 12 hours treatment in HT29 (more CD44). Such phenomenon was not observed in HCT15 (less CD44) after a 6 hours or a 12 hours treatment of HA-dye. **EXAMPLE 3: Synthesis of HA-Celecoxib conjugate** (comparative example)

Procedure

1. 100 mg HA (10K-700KDa) were dissolved in 25 ml DD water.
2. Tetrabutylammonium hydroxide (TBA-OH) 0.8 eq was added into HA solution and stirred for 16 hours.
3. Dried the solution and the HA-TBA white solid was acquired.
4. HA-TBA 40 mg was dissolved in 1 ml DD water and then EDC 30 mg and NHS powder 18 mg were added into the solution and stirred at room temperature for 5 minutes.
5. Celecoxib 4 mg was dissolved in 2 ml dimethylsulfoxide (DMSO) solution.
6. This mixture (HA-TBA, EDC, NHS and Celecoxib) was stirred at room temperature for 72 hours.
7. The mixture was dialyzed for 1 day against that a ratio of DMSO and DD water is 2 to 1 by using dialyzer bag (MWCO: 1200~1400) and changed the solution three times.
8. The mixture was then dialyzed for 2 days against 0.3 M NaCl by using dialyzer bag (MWCO: 1200~1400) and changed the solution two times a day.
9. HA-Celecoxib powder was acquired by dehydration through freeze dryer from HA-Celecoxib solution.

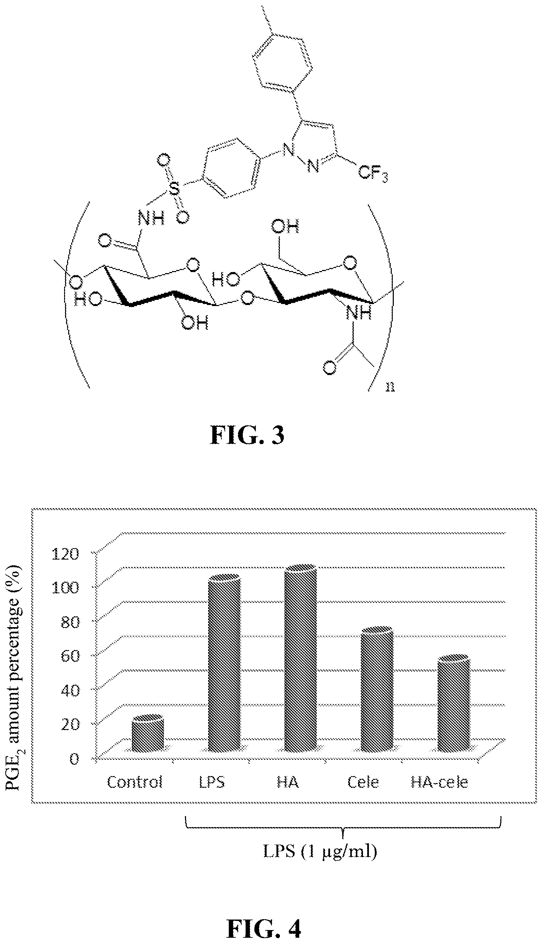

Result: Fig. 3 shows the structure of HA-Celecoxib conjugate.

**EXAMPLE 4: *In vitro* anti-inflammatory effects of HA-Celecoxib on RAW 264.7 cells** (comparative example)

Procedure

1. Raw 264.7 cells ($1 \times 10^6$ cells/well, 1000 $\mu$L) were incubated in a 24-well culture plate in the presence of 5% $CO_2$ at 37°C for 24 hours.
2. The medium was changed to DMEM containing 10% FBS, and the cells were treated with various concentrations of compounds (100 nM Celecoxib, HA-Celecoxib (equal to 100 nM Celecoxib), and 4.9 $\mu$g/ml HA) for 4 hours followed by 1 $\mu$g/mL of lipopolysaccharide (LPS) treatment for 24 h.
3. Cell media were collected, and used for measuring $PGE_2$.
4. Nunc-Immuno 96-well plates were coated with goat polyclonal anti-mouse IgG secondary antibody.
5. Aliquots of cell media were added to the immune plate with primary $PGE_2$ monoclonal antibody and a tracer $PGE_2$-acetylcholinesterase overnight at room temperature in the dark.
6. The next day, the wells were aspirated, washed with 100 $\mu$L wash buffer (Cayman Chemical, Ann Arbor, MI, USA) five times.
7. 200 $\mu$L of Elman's reagent was added in each well, and incubated 60-90 minutes at room temperature out of direct light.
8. Gentle rotating/shaking was used to decrease the time required for color development.
9. Absorbance was read at 412 nm.
10. The $PGE_2$ concentration of each sample was calculated from a $PGE_2$ standard curve.
11. The $PGE_2$ amount percentage was as a function of the $PGE_2$ level of each sample divided by the one of the control group which was LPS-treated, then multiplied this value by 100.
12. The actual amount of $PGE_2$ was estimated to be 1370 ng/ ml for the control group which was LPS-treated.

Result: As shown in Fig. 4, inflammation was successfully induced by LPS as the index is the amount of $PGE_2$. Drug-only group (Celecoxib) has treatment effect; however, when HA conjugated with Celecoxib (HA-cele), it has better treatment effect than drug only.

**EXAMPLE 5: Synthesis of HA-ADH-Budesonide conjugate**

Procedure

Synthesis of HA-ADH:

1. HA (50 mg) was dissolved in water to give a concentration of 4 mg/ml.
2. 5-fold excess (114.8 mg) of ADH was added into the solution.
3. The pH of the reaction mixture was adjusted to 4.75 by addition of 0.1 NHCl.
4. Next, 1 equiv. (25.1 mg) of EDC was added in solid form. The pH of the reaction mixture was maintained at 4.75 by addition of 0.1 N HCl.
5. After 15 minutes reacting, the reaction was quenched by addition of 0.1 N NaOH to adjust the pH of reaction mixture to 7.0.

6. The reaction mixture was then transferred to pretreated dialysis tubing (Mw cutoff 3500) and dialyzed exhaustively against 100 mM NaCl, then 25% EtOH /water 4 cycles and finally water. The solution was then filtered through 0.2 μm cellulose acetate membrane, flash frozen, and lyophilized.

7. The substitution degree of ADH was measured by 1 H NMR.

Synthesis of HA-ADH-Budesonide:

1. HA-ADH-Budesonide succinate was formed by chemical conjugation of the carboxyl group of Budesonide succinate with the amine group of HA-ADH using EDC and NHS.

2. 7.4 mg Budesonide succinate was dissolved in 1 ml DMSO and reacted with 1 ml pure water containing EDC 14.5 μmol and NHS 14.5 μmol for 5 minutes.

3. After 5 minutes, the mixed solution was added dropwise into 8 ml Water: DMSO=1:1(v/v) cosolvent containing 20 mg HA-ADH

4. The mixed solution was stirred 24 hours at room temperature (about 30°C).

5. After 24 hours, the solution was dialyzed 3 days with 0.3 M NaCl and pure water alternately using MWCO 12000-14000 dialysis bag.

6. The solution was then freeze-drying 2 days and stored at 4°C.

7. Finally the degree of substitution was determined by 1H-NMR.

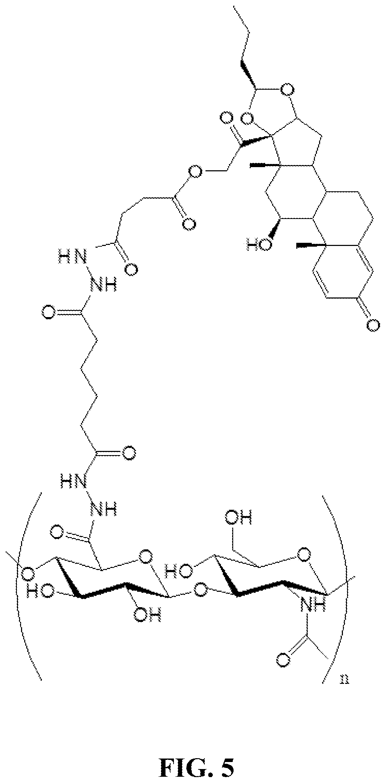

Result: Fig. 5 shows the structure of HA-ADH-Budesonide conjugate.

## EXAMPLE 6: *In vitro* anti-inflammatory effects of HA-ADH-Budesonide on RAW 264.7 cells

Procedure

1. Raw 264.7 cells were suspended in culture medium without phenol red (Gibco-BRL, Vienna, Austria) and adjusted to $10^6$ cells/ml, and treated with 10 μM Budesonide, 378μg/ml HA and HA-ADH-Budesonide (equal to 10 μM Budesonide).

2. After treatment with drugs for 4 hours, 1 μg/mL LPS was added into medium for 24 hours.

3. Cells treated with medium only served as a negative control group and treated with LPS (1 μg/mL) as a positive control group.

4. The culture supernatants were removed subsequently and the nitric oxide were accumulated for the measurement of iNOS activity by the Griess-reaction.

5. 100 μl Griess reagent (1% sulfanilamide, 0.1% naphthlethylenediamine dihydrochloride in 2.5% phosphoric acid) was added into 100 μl culture supernatant and the color development was assessed at 550 nm with a ELISA microplate reader (SpectraMax® M2e Multimode Plate Reader, Molecular Devices, USA).

6. Standard curves were generated with a serial dilution of sodium nitrite dissolved in culture medium which is phenol red free.

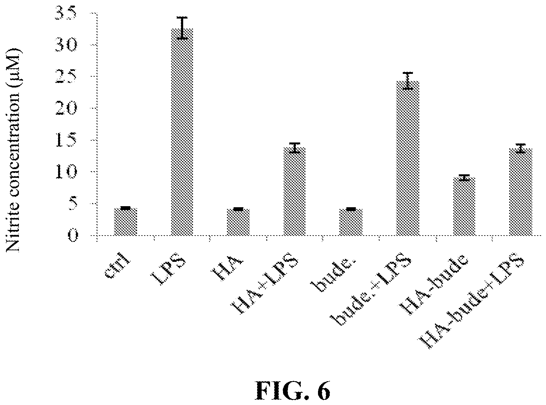

Result: As shown in Fig. 6, when comparing with groups LPS-only, HA+LPS, bude.+LPS, and HA-bude+LPS ("HA-bude" stands for "HA-ADH-Budesonide"), groups HA+LPS and HA-bude+LPS have treatment effect. The inflammation index herein is nitrite.

## EXAMPLE 7: Synthesis of HA-ADH-Fexofenadine conjugate

Procedure

1. HA-ADH-Fexofenadine was synthesized by chemical conjugation of the carboxyl group of Fexofenadine with the amine group of HA-ADH using EDC and NHS.

2. 7.8 mg Fexofenadine was dissolved in 1 ml DMSO and reacted with 1 ml pure water containing 73.3 μmol EDC and 73.9 μmol NHS for 5 minutes.

3. After 5 minutes, the mixed solution was added dropwise into 8 ml water: DMSO=1:1(v/v) cosolvent containing 20 mg HA-ADH

4. The mixed solution was stirred for 24 hours at room temperature (about 30°C).

5. After 24 hours, the solution was dialyzed 3 days with 0.3 M NaCl and water alternately using MWCO 12000-14000 dialysis bag serially.

6. The solution was then freeze-drying 2 days and stored at 4°C.

7. Finally the degree of substitution was determined by 1H-NMR.

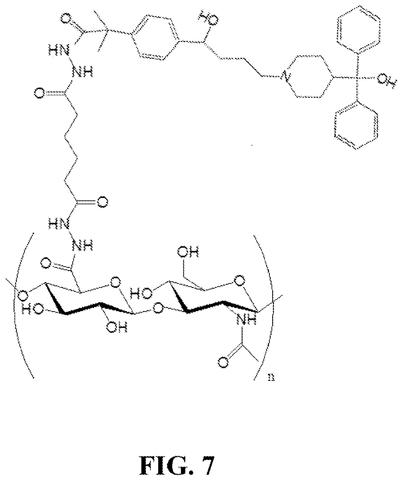

Result: Fig. 7 shows the structure of HA- ADH-Fexofenadine conjugate.

## EXAMPLE 8: *In vitro* anti-inflammatory effects of HA-ADH-Fexofenadine on RAW 264.7 cells

Procedure

1. Raw 264.7 cells ($1 \times 10^6$ cells/well, 1000 μL) were incubated in a 24-well culture plate in the presence of 5% $CO_2$ at 37°C for 24 hours.

2. The medium was changed to DMEM containing 10% FBS, and the cells were treated with various concentrations of compounds (100 $\mu$M Fexofenadine, HA-ADH-Fexofenadine (equal to 100 $\mu$M Fexofenadine), and 2.96 mg/ml HA) for 4 hours followed by 1 $\mu$g/mL of LPS treatment for 24 h.

3. Cell media were collected, and used for measuring $PGE_2$.

4. Nunc-Immuno 96-well plates were coated with goat polyclonal anti-mouse IgG secondary antibody.

5. Aliquots of cell media were added to the immune plate with primary $PGE_2$ monoclonal antibody and a tracer $PGE_2$-acetylcholinesterase overnight at room temperature in the dark.

6. The next day, the wells were aspirated, washed with 100 $\mu$L wash buffer five times.

7. 200 $\mu$L of Elman's reagent was added in each well, and incubated 60-90 minutes at room temperature out of direct light.

8. Gentle rotating/shaking was used to decrease the time required for color development.

9. Absorbance was read at 412 nm.

10. The $PGE_2$ concentration of each sample was calculated from a $PGE_2$ standard curve.

11. The $PGE_2$ amount percentage was as a function of the $PGE_2$ level of each sample divided by the one of the control group which was LPS-treated, then multiplied this value by 100.

12. The actual amount of $PGE_2$ was estimated to be 1370 ng/ml for the control group which was LPS-treated.

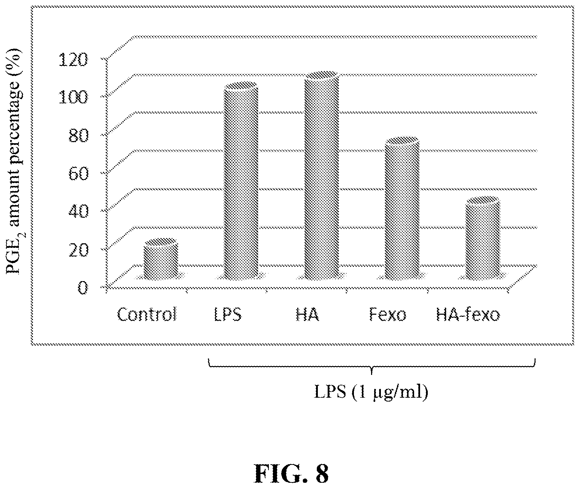

Result: As shown in Fig. 8, drug-only group (Fexofenadine) has treatment effect; however, when HA conjugated with Fexofenadine (HA-fexo, "HA-fexo" stands for "HA-ADH-Fexofenadine"), it has better treatment effect than drug only.

**EXAMPLE 9: Synthesis of HA- ADH-Prednisolone conjugate**

Procedure

1. Synthesis of Prednisolone 21-hemiester:

2. A solution of Prednisolone (1 g; 2.77 mmol) and succinic anhydride (1.125 g; 12.55 mmol) in pyridine (8 mL) was stirred at room temperature.

3. After 24 h, the reaction mixture was poured onto a mixture of ice (25 g), water (25 mL) and conc. HCl (10 mL).

4. The separated crystals were collected by filtration, washed with water, dried, recrystallized from toluene and dried overnight again.

5. The prepared hemiester was characterized by 1H-NMR spectroscopy.

Synthesis of HA-ADH-Prednisolone hemiester:

1. SOLUTION 1: EDC 73.3 $\mu$mol (14 mg) and NHS 73.9 $\mu$mol (8.5 mg) were added in 1 ml water.

2. SOLUTION 2: Prednisolone hemiester 14.5 $\mu$mol (6.4 mg) was dissolved in 1 ml DMSO.

3. SOLUTION 3: HA-ADH (D.S=31%) 20 mg (ADH:14.5 $\mu$mol) was dissolved in 8 ml cosolvent (water: DMSO=1:1).

4. Mix SOLUTION 1 & 2, and stir 5 minutes to activate carbonyl group on Prednisolone hemiester.

5. After 5 minutes, the mixture was added dropwise (1ml/min) to SOLUTION 3 under stirring mixing.

6. The mixed solution was stirred 24 hours at room temperature (about 30°C).

7. After 24 hours, the solution was dialyzed 3 days with 0.3 M NaCl/pure water alternately using MWCO 12000-14000 dialysis bag.

8. The solution was then freeze-drying 2 days.

9. Finally the degree of substitution of HA-ADH-Pred was determined by 1H-NMR.

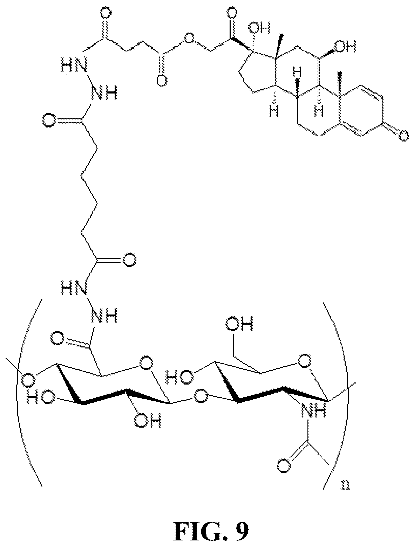

Result: Fig. 9 shows the structure of HA-ADH-Prednisolone conjugate.

**EXAMPLE *10: In vitro* anti-inflammatory effects of HA-ADH-Prednisolone on RAW 264.7 cells**

1. Raw 264.7 cells ($1 \times 10^6$ cells/well, 1000 $\mu$L) were incubated in a 24-well culture plate in the presence of 5% $CO_2$ at 37°C for 24 hours.

2. The medium was changed to DMEM containing 10% FBS, and the cells were treated with various concentrations of compounds (86.7 $\mu$M Prednisolone, HA-ADH-Prednisolone (equal to 86.7 $\mu$M Prednisolone), and 70 $\mu$g/ml HA) for 4 hours followed by 1 $\mu$g/mL of LPS treatment for 24 hours.

3. Cell media were collected, and used for measuring $PGE_2$.

4. Nunc-Immuno 96-well plates were coated with goat polyclonal anti-mouse IgG secondary antibody.

5. Aliquots of cell media were added to the immune plate with primary $PGE_2$ monoclonal antibody and a tracer $PGE_2$-acetylcholinesterase overnight at room temperature in the dark.

6. The next day, the wells were aspirated, washed with 100 $\mu$L wash buffer five times.

7. 200 $\mu$L of Elman's reagent was added in each well, and incubated 60-90 minutes at room temperature out of direct light.

8. Gentle rotating/shaking was used to decrease the time required for color development.

9. Absorbance was read at 412 nm.

10. The $PGE_2$ concentration of each sample was calculated from a $PGE_2$ standard curve.

11. The $PGE_2$ amount percentage was as a function of the $PGE_2$ level of each sample divided by the one of the control group which was LPS-treated, then multiplied this value by 100.
12. The actual amount of PGE$_2$ was estimated to be 1370 ng/ml for the control group which was LPS-treated.

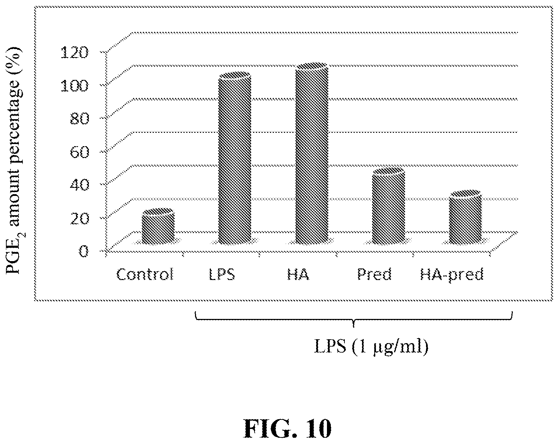

Result: As shown in Fig. 10, drug-only group (Prednisolone) has treatment effect; however, when HA conjugated with Prednisolone (HA-pred, "HA-pred" stands for "HA-ADH-Prednisolone"), it has better treatment effect than drug only.

**EXAMPLE *11: In vivo* treatment effect for Rheumatoid arthritis (RA) by HA-ADH-Prednisolone**

Procedure

1. Thirty of eight weeks male Sprague-Dawely rats (BioLASCO Taiwan Co., Ltd.) were prepared. Rats were divided into 4 groups randomly (n=9 for vehicle and Prednisolone group, n=6 for HA-ADH-Prednisolone and HA group). Every two rats were placed into one cage in rodent animal facility in Institute of Taiwan Animal Technology.
2. Inject 0.1 mL of CFA (Complete Freund's Adjuvant, 10 mg/mL, Chondrex Inc.) containing 10 mg/mL of heat-killed mycobacterium into subcutaneously at a footpad of right hind limb.
3. The needle should be inserted just under the skin of the footpad pointing toward the ankle: this maximizes delivery of the adjuvant to the draining popliteal lymph nodes.
4. Severe and acute inflammation is observed within 30 minutes of injection, peaks within 3 to 4 days, and often persists for 20 to 25 days.
5. Rat were treated at day 14 after induction of the Adjuvant-Induced Arthritis (AIA) by intravenous injection of Prednisolone or HA-Prednisolone (both 10 mg/kg), or saline as a control.
6. Rats were sacrificed at day 20 after treatment and tissues were isolated hereafter.

Arthritis Measurements:

Paw thickness and Ankle circumference: Paw thickness determined by measuring paw diameter using a caliper, such as Mitsutoyo digimatic caliper. Ankle circumference determined by measuring 2 perpendicular diameters, the latero-lateral diameter (a) and the anteroposterior diameter (b), with a digital caliper and using the following formula:

$$\text{circumference} = 2\pi(\sqrt{(a^2+b^2)/2})$$

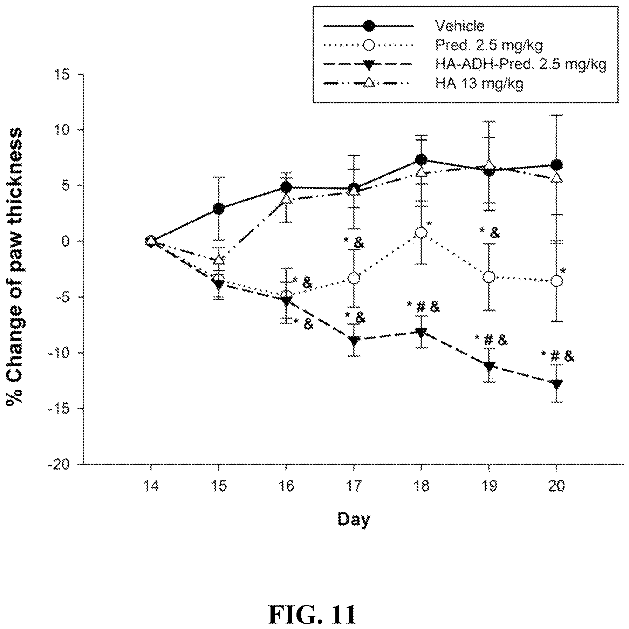

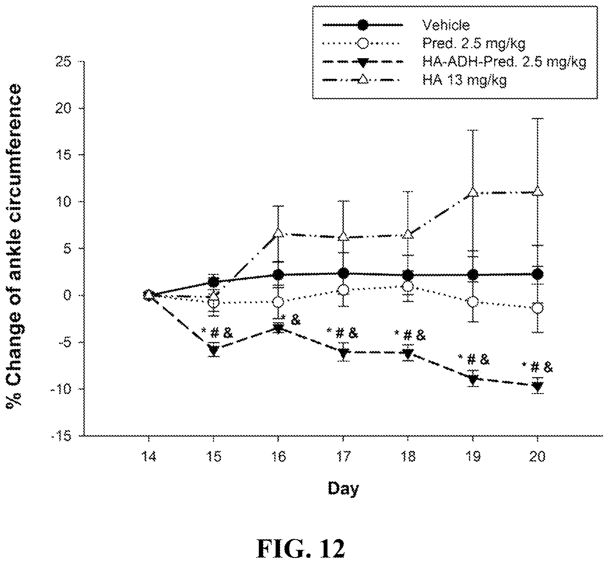

Result: As shown in Fig. 11, RA has been successfully induced. At the beginning of the date administering drug (day 14), HA-only group has almost the same trend comparing with control group (vehicle) in the kind of index as change of paw thickness. The drug-only group (Prednisolone) showed the treatment effect. However, when drug conjugated with HA (HA-ADH-Pred group), its treatment effect was even better than the drug only, and has statistically significance. Also, as shown in Fig. 12, HA only even enhance RA symptom in this kind of index (change of ankle circumference); however, when drug conjugated with HA (HA-ADH-Pred group), its treatment effect was better than the drug only, and has statistically significance.

**Claims**

1. A compound consisting of a conjugate from a glycosaminoglycan and an active compound, wherein the active compound is conjugated by means of a functional group to a carboxylic group of the glycosaminoglycan or a salt thereof to form a covalent conjugation, and the active compound is selected from a group consisting of fexofenadine, budesonide succinate, and prednisolone succinate.

2. The compound according to claim 1, wherein the covalent conjugation is a direct conjugation by means of an ester bond.

3. The compound according to claim 1, wherein the active compound is indirectly conjugated to the carboxylic group of the glycosaminoglycan through a linker.

4. The compound according to claim 3, wherein the linker is selected from a group consisting of adipic dihydrazide, polypeptide, peptide, lipid, amino acid and linear or branched, aliphatic, aromatic or araliphatic C2-C20 dicarboxylic acids.

5. The compound according to claim 1, wherein the glycosaminoglycan is hyaluronic acid.

6. The compound according to claim 5, wherein the hyaluronic acid has an average molecular weight comprised in the range from 5 kDa to 2000 kDa.

7. A compound for use in the treatment of acute or chronic inflammation, wherein the compound is according to claim 1.

8. The compound for use in the treatment of acute or chronic inflammation according to claim 7, wherein the inflammation is induced or caused by infection, injury, autoimmune disease, or allergy.

9. The compound for use in the treatment of acute or chronic inflammation according to claim 8, wherein the autoimmune disease is Rheumatoid arthritis.

10. A compound consisting of a conjugate from a glycosaminoglycan and an active compound according to claim 1 for use in the treatment of inflammation, wherein a therapeutically effective amount of said compound is administered to a subject in need thereof.

11. The compound for use in the treatment of inflammation according to claim 10, wherein the inflammation is induced or caused by infection, injury, autoimmune disease, or allergy.

12. A pharmaceutical composition, comprising at least one conjugate from a glycosaminoglycan and an active compound according to claim 1 in combination with at least one excipient and/or diluent.

13. A pharmaceutical composition for use in the treatment of inflammation, wherein the pharmaceutical composition is according to claim 12.

14. The pharmaceutical composition for use in the treatment of inflammation according to claim 13, wherein the inflammation is induced or caused by infection, injury, autoimmune disease, or allergy.

15. The pharmaceutical composition according to claim 12, wherein the pharmaceutical composition is designed for oral or injectable administration.

**Patentansprüche**

1. Eine Verbindung bestehend aus einem Konjugat aus einem Glycosaminoglycan und einer aktiven Verbindung, wobei die aktive Verbindung mittels einer funktionellen Gruppe an eine Carboxylgruppe des Glycosaminoglycans oder eines Salzes davon konjugiert ist, um eine kovalente Konjugation zu bilden, und die aktive Verbindung ausgewählt ist aus einer Gruppe bestehend aus Fexofenadin, Budesonidsuccinat und Prednisolonsuccinat.

2. Die Verbindung gemäß Anspruch 1, wobei die kovalente Konjugation eine direkte Konjugation mittels einer Esterbindung ist.

3. Die Verbindung gemäß Anspruch 1, wobei die aktive Verbindung indirekt über einen Linker an die Carboxylgruppe des Glycosaminoglycans konjugiert ist.

4. Die Verbindung gemäß Anspruch 3, wobei der Linker ausgewählt ist aus einer Gruppe bestehend aus adipischem Dihydrazid, Polypeptid, Peptid, Lipid, Aminosäure und linearen oder verzweigten, aliphatischen, aromatischen oder araliphatischen C2-C20-Dicarbonsäuren.

5. Die Verbindung gemäß Anspruch 1, wobei das Glykosaminoglykan Hyaluronsäure ist.

6. Die Verbindung gemäß Anspruch 5, wobei die Hyaluronsäure ein durchschnittliches Molekulargewicht im Bereich von 5 kDa bis 2000 kDa aufweist.

7. Eine Verbindung zur Verwendung bei der Behandlung einer akuten oder chronischen Entzündung, wobei die Verbindung gemäß Anspruch 1 ist.

8. Die Verbindung zur Verwendung bei der Behandlung einer akuten oder chronischen Entzündung gemäß Anspruch 7, wobei die Entzündung durch eine Infektion, Verletzung, Autoimmunerkrankung oder Allergie induziert oder verursacht ist.

9. Die Verbindung zur Verwendung bei der Behandlung einer akuten oder chronischen Entzündung gemäß Anspruch 8, wobei die Autoimmunerkrankung Rheumatoide Arthritis ist.

10. Eine Verbindung bestehend aus einem Konjugat aus einem Glykosaminoglykan und einer aktiven Verbindung gemäß Anspruch 1 zur Verwendung bei der Behandlung einer Entzündung, wobei eine therapeutisch wirksame Menge der Verbindung an ein Subjekt verabreicht wird, welches diese benötigt.

11. Die Verbindung zur Verwendung bei der Behandlung einer Entzündung gemäß Anspruch 10, wobei die Entzündung durch eine Infektion, Verletzung, Autoimmunerkrankung oder Allergie induziert oder verursacht ist.

12. Eine pharmazeutische Zusammensetzung umfassend mindestens ein Konjugat aus einem Glycosaminoglycan und einer aktiven Verbindung gemäß Anspruch 1 in Kombination mit mindestens einem Trägerstoff und/oder Verdünnungsmittel.

13. Eine pharmazeutische Zusammensetzung zur Verwendung bei der Behandlung einer Entzündung, wobei die pharmazeutische Zusammensetzung gemäß Anspruch 12 ist.

14. Die pharmazeutische Zusammensetzung zur Verwendung bei der Behandlung einer Entzündung gemäß Anspruch 13, wobei die Entzündung durch eine Infektion, Verletzung, Autoimmunerkrankung oder Allergie induziert oder verursacht ist.

15. Die pharmazeutische Zusammensetzung gemäß Anspruch 12, wobei die pharmazeutische Zusammensetzung zur oralen oder injizierbaren Verabreichung bestimmt ist.

**Revendications**

1. Composé consistant en un conjugué d'un glycosaminoglycane et d'un principe actif, dans lequel le principe actif est conjugué au moyen d'un groupe fonctionnel à un groupe carboxylique du glycosaminoglycane ou d'un sel de celui-ci pour former une conjugaison covalente, et le principe actif est choisi dans le groupe constitué par la féxofénadine, le succinate de budésonide, et le succinate de prednisolone.

2. Composé selon la revendication 1, dans lequel la conjugaison covalente est une conjugaison directe au moyen d'une liaison ester.

3. Composé selon la revendication 1, dans lequel le principe actif est indirectement conjugué au groupe carboxylique du glycosaminoglycane par l'intermédiaire d'un lieur.

4. Composé selon la revendication 3, dans lequel le lieur est choisi dans le groupe constitué par le dihydrazide adipique, un polypeptide, un peptide, un lipide, un acide aminé et un acide dicarboxylique en $C_2$ à $C_{20}$ aliphatique, aromatique ou araliphatique, linéaire ou ramifié.

5. Composé selon la revendication 1, dans lequel le glycosaminoglycane est l'acide hyaluronique.

6. Composé selon la revendication 5, dans lequel l'acide hyaluronique a une masse moléculaire moyenne comprise dans la plage allant de 5 kDa à 2000 kDa.

7. Composé pour son utilisation dans le traitement d'une inflammation aiguë ou chronique, dans lequel le composé est selon la revendication 1.

8. Composé pour son utilisation dans le traitement d'une inflammation aiguë ou chronique selon la revendication 7, dans lequel l'inflammation est induite ou provoquée par une infection, une blessure, une maladie auto-immune, ou une allergie.

9. Composé pour son utilisation dans le traitement d'une inflammation aiguë ou chronique selon la revendication 8, dans lequel la maladie auto-immune est la polyarthrite rhumatoïde.

10. Composé consistant en un conjugué d'un glycosaminoglycane et d'un principe actif selon la revendication 1, pour son utilisation dans le traitement d'une inflammation, dans lequel une quantité efficace du point de vue thérapeutique dudit composé est administrée à un sujet en ayant besoin.

11. Composé pour son utilisation dans le traitement d'une inflammation selon la revendication 10, dans lequel l'inflammation est induite ou provoquée par une infection, une blessure, une maladie auto-immune, ou une allergie.

12. Composition pharmaceutique comprenant au moins un conjugué d'un glycosaminoglycane et d'un principe actif selon la revendication 1 en combinaison avec au moins un excipient et/ou diluant.

13. Composition pharmaceutique pour son utilisation dans le traitement d'une inflammation, dans laquelle la composition pharmaceutique est selon la revendication 12.

14. Composition pharmaceutique pour son utilisation dans le traitement d'une inflammation selon la revendication 13, dans laquelle l'inflammation est induite ou provoquée par une infection, une blessure, une maladie auto-immune, ou une allergie.

15. Composition pharmaceutique selon la revendication 12, dans laquelle la composition pharmaceutique est conçue pour être administrée par voie orale ou par injection.

**FIG. 1**

**FIG. 2A**

**FIG. 2B**

FIG. 2C

FIG. 2D

**FIG. 3**

**FIG. 4**

**FIG. 5**

**FIG. 6**

**FIG. 7**

**FIG. 8**

**FIG. 9**

**FIG. 10**

**FIG. 11**

**FIG. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61871352
- WO 9409811 A
- WO 9945942 A

- US 8192744 B
- US 2004157810 A

### Non-patent literature cited in the description

- **GUNTHERT et al.** *A new variant of glycoprotein CD44 confers metastatic potential to rat carcinoma cells,* 1991, vol. 5;65 (1), 13-24
- **DIANHUA JIANG.** Hyaluronan in Tissue Injury and Repair. *Annu. Rev. Cell Dev. Biol.,* 2007, vol. 23, 435-61
- **SHIGEKI KATOH et al.** Galectin-9 Inhibits CD44-Hyaluronan Interaction and Suppresses a Murine Model of Allergic Asthma. *American Journal of Respiratory and Critical Care Medicine,* 01 July 2007, vol. 176 (1), 27-35
- **YUNG S ; CHAN TM.** The Role of Hyaluronan and CD44 in the Pathogenesis of Lupus Nephritis. *Autoimmune Dis.,* 2012, vol. 2012, 9
- **PATRICK J. MOTT.** CD44 Antibodies and Immune Thrombocytopenia in the Amelioration of Murine Inflammatory Arthritis. *PLoS One,* 2013, vol. 8 (6), e65805
- **JOHN A. ICE.** Genetics of Sjögren's syndrome in the genome-wide association era. *J Autoimmun.,* August 2012, vol. 39 (1-2), 57-63

- **ROMAN FISCHER.** Discovery of Candidate Serum Proteomic and Metabolomic Biomarkers in Ankylosing Spondylitis. *Mol Cell Proteomics,* February 2012, vol. 11 (2), M111.013904
- **ESTESS P et al.** Functional activation of lymphocyte CD44 in peripheral blood is a marker of autoimmune disease activity. *J Clin Invest.,* 15 September 1998, vol. 102 (6), 1173-82
- **WEN-QING LI.** Psoriasis, psoriatic arthritis and increased risk of incident Crohn's disease in US women. *Ann Rheum Dis.,* July 2013, vol. 72 (7), 1200-5
- **HENRY S. SU.** Vasculitis: Molecular Imaging by Targeting the Inflammatory Enzyme Myeloperoxidase. *Radiology,* January 2012, vol. 262 (1), 181-90
- **M. B. OWLIA.** Behcet's Disease: New Concepts in Cardiovascular Involvements and Future Direction for Treatment. *ISRN Pharmacol,* 2012, vol. 2012, 760484
- **ONISHI HIRAKU et al.** In vivo evaluation of chondroitin sulfate-glycyl-prednisolone for anti-arthritic effectiveness and pharmacokinetic characteristics. *International journal of pharmaceutics,* 2013, vol. 456 (1), 113-120